# EUROPEAN PATENT APPLICATION

(11) **EP 4 163 874 A1**
(43) Date of publication of application: **12.04.2023**
(21) Application number: 21306408.2
(22) Date of filing: 06.10.2021
(51) Int. Cl.: G06T 15/08, A61B 34/20

(54) **METHOD AND DEVICE FOR AN IMPROVED DISPLAY IN A MANUAL OR A ROBOT ASSISTED INTERVENTION IN A REGION OF INTEREST OF THE PATIENT**

(71) Applicant: MinMaxMedical, 38400 Saint-Martin-d'Hères (FR)
(72) Inventor: CLERE, Arnaud, 38400 Saint Martin d'Heres (FR); KAUFMANN, Alain, 38400 Saint Martin d'Heres (FR)
(74) Representative: Regimbeau

(57) **Abstract**

The invention concerns a computer implemented method for processing images comprising the following steps:
a) obtaining (100) a 3D image (I-3D) of a region of interest (ROI) including an anatomical region of a patient;
b) obtaining (200) at least one projection (P1) of the region of interest (ROI) of the patient (3), said projection (P1) being a 2D overall image of said region of interest (ROI) and showing information of a first type about said region according to a point of view (Pov);
c) obtaining (300) at least one slice (S) from the 3D image (I-3D), said slice (S) comprising a point of interest (POI) in said region of interest (ROI), said obtained slice containing information about the region of interest (ROI) of a second type around the point of interest (POI);
d) computing (400) a composite 2D image wherein a portion (p) of one of the 2D overall image containing information about the point of interest (POI) is replaced by the projection of a corresponding portion of the obtained slice (S) from the same point of view (Pov) so that information of the second type is accurately arranged relatively to information of the first type present in the 2D overall image.

## Description

### FIELD OF THE INVENTION

The invention concerns a method, a computer implemented method and a system for allowing appropriate display for assisting a manual or a robot assisted intervention in a region of interest of the patient. In particular, the invention concerns the manual or robot-assisted navigation of a medical instrument and concerns a computer implemented method, a method and a system for allowing appropriate display of the position of such medical instrument with respect to anatomical features in the region of interest of a patient during the navigation.

### BACKGROUND OF THE INVENTION

During a medical intervention assisted by surgical navigation or using a robot, a practitioner may have to insert an instrument in a region of interest of a patient and aim to an anatomical feature, for example a patient's vertebra pedicle.

To do so, the practitioner must be able to visualize the region of interest, the anatomical feature, and the instrument. However, this is often complicated for at least the following reasons.

In standard navigation systems, a 3D image of the patient is generally used, and the instrument tip and/or axis is usually represented on slices of the 3D image passing through said tip and/or axis. Said slices are moving in real-time together with the position of the instrument which is tracked by the navigation system with respect to the 3D image using well known registration and calibration methods. When the instrument is not in the desired position, the user needs to interpret the slice to decide to move the instrument towards a given direction. This is not easy. Several slices are usually computed in several planes and displayed on the same screen, to suggest a 3D representation, but the issues mentioned above remain and the spatial complexity is higher.

Human factors limit the surgeon's ability to translate multiple 2D information into a multi-dimensional gesture corresponding to the position and orientation of the instrument during the intervention.

For facilitating manual navigation, or controlling robot-assisted navigation, it is possible to define a target and perform a planning in the 3D image as a first step, and to display a fixed slice computed in the 3D image passing through the tip and axis of the planned position of the instrument, and then to project the real position of the instrument on the slice. However, until the instrument reaches exactly the planned target position, the image does not show where the real tool is and this can be misleading and dangerous.

### SUMMARY OF THE INVENTION

The invention selects two types of information according to precise criteria and combine them in a unique way so as to provide complementary information to the practitioner that is more accurate and non-ambiguous and permits to respond at least to the above-mentioned drawbacks of the known art.

To this end, according to a first aspect, the invention concerns a computer implemented method comprising the following steps:
a) obtaining a 3D image of a region of interest including an anatomical region of a patient;
b) obtaining at least one projection of the region of interest of the patient, said projection being a 2D overall image of said region of interest and showing information of a first type about said region according to a point of view;
c) obtaining at least one slice from the 3D image, said slice comprising a point of interest in said region of interest, said obtained slice containing information about the region of interest of a second type around the point of interest;
d) computing a composite 2D image wherein a portion of one of the 2D overall image containing information about the point of interest is replaced by the projection of a corresponding portion of one of the obtained slice from the same point of view so that information of the second type is accurately arranged relatively to information of the first type present in the 2D overall image.

The invention according to the first aspect, may be complemented by one or several of the following features, alone or in combination.

The method further comprises a step of tracking a medical instrument into the region of interest, said instrument having a main axis.

The obtained slice is a slice wherein said medical instrument main axis is tracked into the region of interest. Thus, it is possible to follow the navigation of the instrument into the information of the second type.

The slice is oriented along a plane orthogonal to a direction of the projection. The slice passes through the main axis of the medical instrument, wherein the 3D image is associated with a reference frame comprising three cartesian axes X, Y, Z and wherein the slice passes through one of these cartesian axes, such that the slice contains the instrument main axis and has a direction parallel to the chosen cartesian axis. The direction is named pseudo sagittal, pseudo axial or pseudo coronal depending on the selected axis X, Y or Z. Thus, it permits one degree of freedom on the position of the slice and optionally one degree of freedom on the orientation in addition to several degrees of freedom on the portion of the slice.

The at least one slice is parallel to and at a fixed distance from a plane comprising a display configured to display the composite 2D image, the point of view being located on the plane comprising the display said display being tracked during its movement. Thus, it permits no degree of freedom on the selection of the slice, but moving the display allows to physically explore the region of interest, and degrees of freedom can be given to the user regarding the portion of the slice to provide a suitable balance of the two types of information.

The portion of the slice has a width depending on a distance between the instrument main axis and a target point and/or a height defined along the main axis of the instrument.

The point of interest belongs to the main axis of the medical instrument, the region of interest corresponding to a region in which the medical instrument has to be inserted or has been inserted according to this axis Thus, it permits two degrees of freedom on the orientation of the slice in addition to degrees of freedom on the extent of the portion around the point of interest.

The computation of the slice comprises the determination of a plane in which the whole instrument axis is located. Thus, it is possible to display in the composite image, the whole instrument.

The computation of the composite 2D image comprises the projection on said composite 2D image, of either the main axis of an instrument the slice being centered around this axis, and/or the projection of medical intervention targets. Thus, it is possible to obtain further information on the composite image.

The method comprises a step of selecting a projection among a plurality of projections, each projection being acquired or computed from the 3D image,
- said selection depending on a point of view, said point of view preferably depending of position and/or orientation of a tracked device such as e.g. a medical instrument, relative to the region of interest or an anatomical feature, or
- said selection depending on the point of interest or the region of interest. The portion of the slice is a strip delimited around the axis of the instrument.

The 2D overall image is chosen from the following group: a radiographic image obtained by emission of x-ray to the anatomical region, a Digitally-Reconstructed Radiography, a computed Maximum Intensity Projection, a computed Average Intensity Projection, possibly with enhanced contrast, a computed Minimum Intensity Projection, a computed Locally Minimum, respectively Maximum, Intensity Projection.

The method comprises a step of switching between two points of view, said method implementing the steps b) and d) for taking into account this point of view.

The method comprises a step of switching between two slices, said method implementing the steps c) and d) for taking into account the second slice This switching is preferably triggered when a relation between an axis of a tracked device and the direction of the selected projection being below or equal to a defined threshold. This threshold is, preferably comprised in a range defined between 35° and 45, preferably 45°.

The method further comprises a step of displaying the composite image.

According to a second aspect, the invention concerns a medical navigation system comprising a processor configured for implementing the method of the first aspect of the invention, and at least one display that is connected to or integrated to a medical imaging system for implementing the method according to the first aspect.

By the use of a composition of 2D images providing different information, it is possible to enhance the navigation in a region of interest during a medical intervention.

The method of the invention ensures that all information presented in the composite 2D image always represent their respective geometric positions in the patient accurately, which permits to facilitate the exploration or navigation of an instrument into the patient. Effectively, it allows to present at the same time and in the same reference frame several relevant information to the practitioner. The obtained projection permits to display in general where the instrument navigates into the region of interest relative to key anatomical features, most of which cannot be presented at the same time in any single slice. In the meantime, the obtained slice permits to display in detail the anatomical features immediately surrounding the main axis of the navigated instrument. And both are accurately presented with respect to each other.

In particular, the obtained projection always provides a broad view of the patient's anatomy, even when the navigated instrument is not in the region of interest, which allows the surgeon to quickly understand the orientation of the overall and composite image with regard to the patient because it presents more information and eliminates some of the ambiguities of slices at once. For example, if the heart is part of the 3D image with surrounding bony structures, such as ribs and spine, it will most often appear on a projection but will be absent from many slices.

Projections do not allow to precisely locate a surgical instrument as the pixels surrounding the navigated instrument may present anatomical features located in planes far away from the instrument. Thus, the slice permits to complement the information present in the projection.

### BRIEF DESCRIPTION OF THE DRAWINGS

Other features and advantages of the invention will appear in the following description. Embodiments of the invention will be described with reference to the drawings, in which
- Figure 1 illustrates a medical navigation system and a display system according to an embodiment of the invention;
- Figures 2 and 3 illustrate a method according to an embodiment of the invention;
- Figures 4, 5a and 5b illustrate composite images obtained with the method according to the invention.

### DETAILED DESCRIPTION OF THE INVENTION

### Medical navigation system

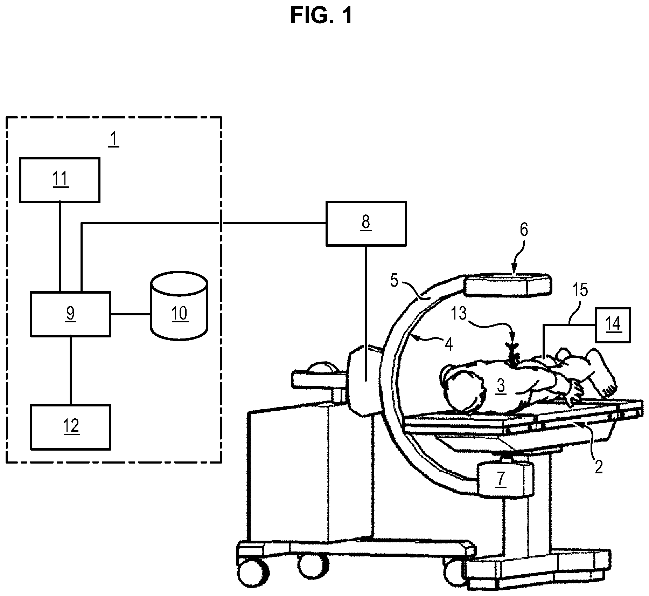

**Figure 1** illustrates a medical navigation system 1 and an operating table 2 on which a patient 3 may be disposed. In particular, the patient 3 may lie on this table 2 for surgical spine procedure for instance. The medical navigation system 1 may also be used in various types of medical interventions, including surgery and interventional radiology.

The medical navigation system 1 may also be linked to or integrated with a medical imaging device 4 for acquiring medical images comprising a detector 6 and an X-ray source 7. The X-ray source 7 and the detector 6 are, in particular, mounted facing each other. This device 4 may be a cone beam computed tomography (CBCT) as represented, or a computed tomography (CT) imaging system both well known in the art. The detector 6 and the X-ray source 7 may be arranged at ends of a C-arm 5 which is preferably rotatably mounted around the operating table 2 for acquiring images according to different positions of the C-arm relative to the patient 3.

The medical imaging device 4 comprises a processing unit 8 (such as a computer) for controlling the device, reconstructing 3D images from acquired 2D images, and sending them to the medical navigation system 1. To this end, the processing unit 8 is connected to the medical navigation system 1 by means of any suitable communication link.

The medical navigation system 1 comprises a storage unit 10 configured for storing at least the acquired and reconstructed images, and a processing unit 9 for processing images, in particular a method for processing images in order to facilitate the navigation of a medical instrument (see hereafter). Furthermore, the navigation system 1 comprises one or several display(s) 11 for the display of processed images or other parameters that the practitioner needs.

By display we mean any suitable device for displaying information to the practitioner: means suitable for augmented reality, virtual reality, computer screen, means for projecting images, etc. In any case, the invention cannot be limited to a particular display.

The navigation system 1 permits, in particular, to facilitate the gesture of the practitioner or control the gesture of a robot (not represented) when he manipulates an instrument inserted into a patient. Thus, for locating the instrument during the navigation, the system 1 comprises or is connected to a tracking system 12 to track the instrument relative to the patient and the images relative to the patient. As known, the tracking system 12 can be an optical tracking system, an electromagnetic tracking system, an ultrasonic tracking system, an inertial measurement unit, a mechanical tracking system, or any combination of those. The tracking system locates the position of a tracker 14 attached to an instrument 15 with respect to a reference tracker 13 attached to a patient anatomical structure.

The instrument is, preferably, a linear instrument such as a trocar, a needle, a drill, a burr or equivalent. Alternatively, the instrument may be a planar instrument with a main axis, such as an oscillating saw with the median axis of the cutting blade. The instrument equipped with a tracker can be navigated free-hand or inserted in a guide held by a robot or actively moved by a robot.

The navigation system 1 permits to explore a region of interest of a patient in which an instrument has to be inserted, or during the insertion, or after an intervention in this region, such as for verification purposes.

### Method

In the following we will describe a method for processing images for assisting a navigated or robot-assisted intervention in a region of interest of the patient aiming to an anatomical feature.

This method uses at least one 3D image of a region of interest of the patient and possibly several 2D projections corresponding to different points of view of the patient (for instance, these points of view are related to different orientations of a CBCT's C-arm during the acquisition). The 3D image can be acquired before the surgery and a well-known registration method can be used to determine a geometric relationship between the reference tracker 13 attached to the patient and the 3D image. The 3D image can also be acquired during the surgery. The 2D projections can be generated from the 3D image or they can be acquired using a 2D X-ray imaging device that is registered with the reference tracker 13. In a preferred embodiment, an intraoperative CBCT made of a motorized C-arm is used to generate a 3D image from a plurality of X-ray projections and some of those X-ray projections are used in the invention, and the intraoperative CBCT is registered with the reference tracker 13.

In the following, we consider that, when a medical instrument is used in the region of interest, that instrument is always accurately tracked relative to a considered representation of the region of interest, this tracking being possible by the use of known method as described, for example in document US20140049629A1.

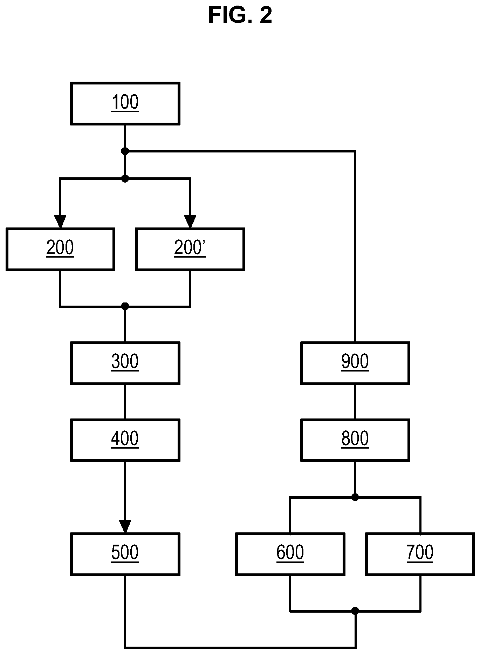

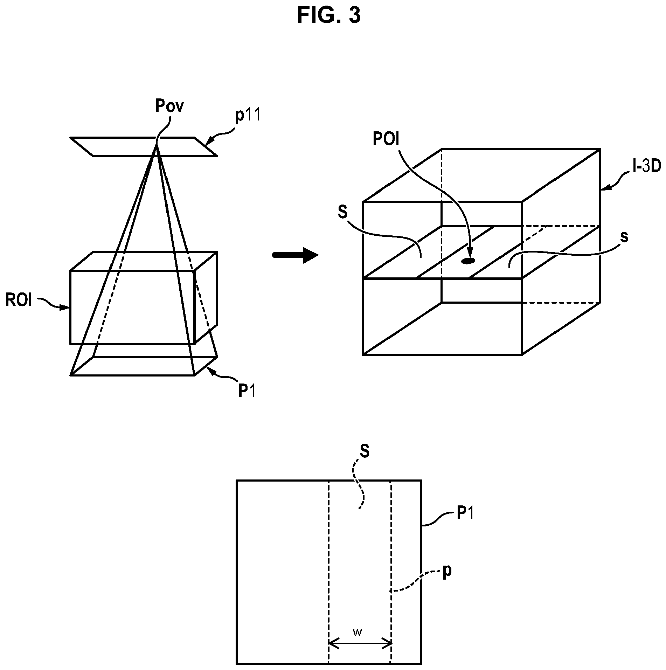

In relation to **figures** 2 and **3****,** the method comprises, as a preliminary step, the obtention of a 3D image I-3D of a region of interest ROI of the patient (step 100).

As known, this 3D image of this region can be obtained from projections acquired according to different positions of the C-arm or CT scanning system in the reference frame of the medical imaging system. In particular, the 3D image is obtained by a tomography reconstruction process of the region. The obtention of the 3D image is well known by the man skilled in the art and will not be described further.

For assisting the intervention in the anatomical region of a patient two types of images are needed. In particular, projections of the region of interest and slices of this region are needed. By region of interest it has to be understood a region having a thickness greater than the patient's thickness *(i.e.,* including a region of the patient along with its environment and corresponding to the whole region traversed by X-rays), a region having a thickness restricted to the patient's anatomy (corresponding to the region traversed by X-rays into the patient), a region having a thickness restricted to a portion of the patient's anatomy (corresponding to the region traversed by X-rays into a portion only of the patient).

The projections provide global information on the region, also referred to as information of a first type while slices provide local information of the region, also referred to as information of a second type.

Also, the method comprises the obtention (step 200) of at least one projection P1 of the region of interest ROI according to a point of view Pov. Such a projection is a 2D image obtained by projecting the 3D anatomy of the patient according to a selected source and direction onto a 2D plane, said source being called the projection's "point of view". These projections provide information of the first type on anatomical features in a region of interest of a patient as they are projecting several 3D voxels onto a single 2D pixel. In particular, these projections provide an overall view of anatomical features of the patient and preferably allow the surgeon to quickly position and orient an instrument in that region with fewer errors. In particular, this projection provides a 2D overall image of the region of interest of the patient.

The projection can be obtained by acquisition on the patient itself or by simulation as described here below.

According to an embodiment, a projection is a radiographic image whose pixels quantify a level of X-ray absorption by the 3D anatomy of the patient according to a X-ray source and direction to the X-ray detector found e.g. in CBCT imaging systems.

According to another embodiment, a projection can be computed from a 3D image to simulate radiographs and are well known as Digitally-Reconstructed Radiographs (DRR) as described in document US7653226. Such DRR avoid having to expose the patient to repeated doses of radiation to generate a specific projection needed. In the case of CT scanner which use 1D X-ray detectors, it may be the only physical solution to present radiographic images to practitioners.

According to another embodiment, a projection can be a well-known Maximum Intensity Projection (MIP) computed by retaining for each 2D pixel the maximum intensity value of all the 3D voxels projected onto it.

The obtained projection (step 200) depends on the most appropriate point of view for the medical intervention. This point of view can be defined automatically when anatomical features can be detected so as to, e.g. maximize the visibility of the spine or follow a targeted axis or plane.

Alternatively, the projection can be selected manually by the practitioner among several projections acquired or pre-computed before the intervention (step 200'). In that case, the practitioner has a limited number of projections each corresponding to a point of view of the region of interest.

As already mentioned, a second type of 2D images named hereafter slice(s) is involved.

In particular, at least one slice is obtained (step 300). Each slice is obtained by computing the intersection of a plane with the 3D image I-3D (the one obtained by tomography reconstruction for instance) of a region of interest of a patient, centered around the point of interest POI in the region of interest. In particular, the obtained slice may be oriented along a plane orthogonal to the direction of the projection. In general, the slice is computed to pass through the main axis of the instrument position determined in the 3D image and through one of the cartesian axis of the image reference frame X, Y or Z, such that the resulting slice contains the instrument axis and has a direction, named pseudo sagittal, pseudo axial or pseudo coronal, depending on the selected axis X, Y or Z. The obtained slice permits to obtain information of the second type on the anatomical region. In particular, and as known, this slice makes it possible to obtain unambiguous local information located in a specified plane of the region of interest.

Once the 2D overall image and the slice obtained, the method combines both information by the computation of a composite 2D image (step 400).

In particular, the method selects a slice (see step 300) of the region of interest and combines it with the projection (see step 200) so that the information contained in the slice corresponds with the information contained in the projection in an accurate way with respect to the geometry and to the point of view of the region of interest.

In particular, that composite 2D image is made from the projection P1 from which a portion is replaced by the projection from the same point of view Pov of a portion p of the computed slice S so that information of the second type, which is local and unambiguous, is displayed in the context of information of the first type, which is global, provided by the projection. Therefore, information of the second type is accurately displayed relatively to information of the first type present in the 2D overall image. In particular, the slice is projected onto the overall image by using the same parameters of projection used to obtain the overall image.

Once the composite 2D image is computed, this composite 2D image is displayed (step 500), preferably in a specific window, on a display such as e.g., computer screens, augmented reality display (using glasses, semi-transparent screen, projectors, etc.), or other display that can be used by a practitioner in order to conduct an intervention on a patient. This composite image is advantageously represented in a window of a screen; Other windows are used to represent standard slices, projections or combined views in other directions.

According to an aspect, the method allows the switching between several visualization modes.

According to an embodiment, the method comprises a step of switching (step 600) between two points of view for visualizing the region of interest. In other words, it permits to switch between a first overall image showing the region of interest from a first point of view to a second overall image showing the region of interest from a second point of view. Then, the method repeats the steps of obtaining the second projection (step 200) and computing (step 400) the composite 2D image with the second overall image. It has to be understood that the slice remains unchanged.

According to an embodiment, the method comprises a step of switching (step 700) between two types of slices. In other words, it permits to switch between a first slice and a second slice, the overall image remaining unchanged. Then the method repeats the steps of obtaining the second slice (step 300) and computing (step 400) the composite 2D image with the second slice. This switching may advantageously be triggered when a relation between an axis of a tracked device (e.g., the linear axis of the device) and the direction of the selected projection being below or equal to a defined threshold. This threshold may be, preferably, comprised in a range defined between 35° and 45, preferably 45°, so that the selected slice is never exactly or closely aligned with the direction that will be used to project it in the step of computing the composite 2D image (step 400).

The above-described method can be implemented in several contexts: to explore a region of interest of a patient, for guiding an instrument. Thus, the slice can be computed according to several criteria depending on the needs of the practitioner, the displayed image toggling accordingly.

According to an embodiment, the slice is such that the point of interest POI belongs to an axis of interest of a medical instrument, the region of interest corresponding to a region in which a medical instrument has to be inserted or has been inserted according to this axis. For instance, the point of interest POI belongs to the main axis of the medical instrument

According to an embodiment, the slice can be one that also contains vectors parallel to a 3D image axis which are usually assimilated to anatomical directions such as anteroposterior direction or lateral direction.

According to another embodiment, the slice can be the one that is parallel to and at a fixed distance from a plane p11 comprising a display configured to display the composite 2D image, the point of view being located on the plane comprising the display, such display being tracked during its movement. Such a display can be a portable screen manipulated by the practitioner and tracked by device 12 to scan through the patient anatomy intuitively since the relation between the physical display and the slice is a simple translation.

Advantageously, the method comprises a step of tracking (step 800) a medical instrument into the region of interest, the instrument having an axis, the obtained slice being a slice wherein the medical instrument is tracked into the region of interest. This tracking is implemented during the navigation into the region of interest. In that case, the selection of a projection depends on a point of view, this point of view preferably depending on the position and/or orientation of a tracked device such as e.g., a medical instrument relative to the region of interest or an anatomical feature.

When a medical instrument is tracked, the obtained slice can be the one that is orthogonal to the medical instrument axis and located at a particular point along the axis, e.g., 1cm before, after, or exactly at the tip of the medical instrument.

Also, the computation of the slice S may comprise the determination of a plane in which a whole instrument (if available) is located during the tracking, the computed slice comprising or intersecting this plane.

According to an embodiment, the computation of the composite 2D image comprises (step 900) the projection of an axis and/or a 3D model of an instrument, and/or the projection of medical intervention targets. The projection of additional objects provides additional information to the practitioner during the intervention. In particular, it provides the possibility to visualize the instrument over the slice and the projection.

According to an embodiment, the selected portion of the obtained slice is a strip defined around an axis of an instrument, or the projection of a parallelogram of fixed dimensions around the axis.

According to another embodiment, the selected portion of the obtained slice is an ellipse around the axis of an instrument in the plane of the obtained projection, or the projection of a cylinder of fixed radius around the axis of the instrument.

According to another embodiment, the selected portion of the obtained slice is a circle or square around the point of interest, that can be the tip of the instrument.

### Examples

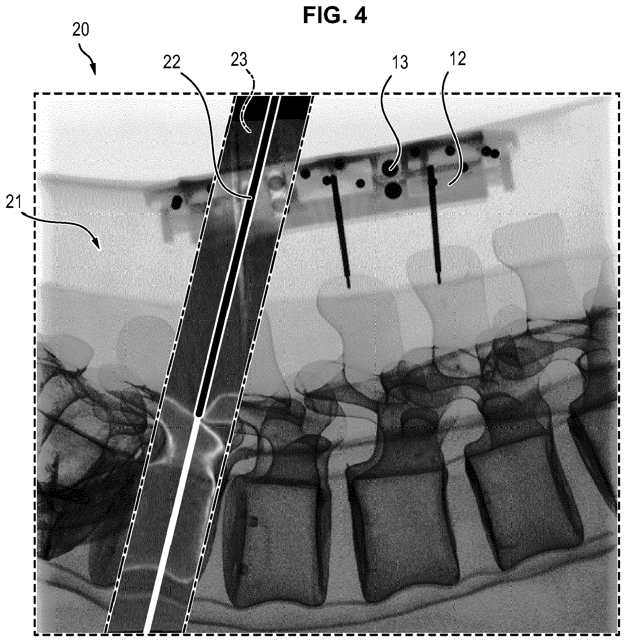

**Figure 4** shows an example of a composite 2D image 20 that can be obtained according to the method here described. This image shows a lateral projection 21 of the lumbar region in which the spine is visible. This projection is selected from a series of projection used to build the 3D image using a CBCT imaging device. This lateral projection provides an overall image of the region in which the practitioner must insert an instrument. On this image 20 a patient fiducial 12 equipped with radio-opaque markers 13 allows to know the pose of the patient in the image (see above).

On this image, all present vertebrae are visible and the surgeon can insert the instrument into one of these vertebra (the fourth entire one from the left of the image) with almost no risk of aiming a wrong vertebra.

On this image the axis 22 of the instrument is projected on the obtained projection, which is always meaningful and useful since it corresponds to a virtual X-ray image of the patient and the instrument.

Around this axis 22 a portion of a slice 23 is computed and projected. According to the illustrated embodiment, the slice is a plane that passes through the axis 22 of the instrument and that also contains the Z cartesian axis of the 3D image reference frame, which creates a pseudo sagittal slice. One can see that anatomical features where the instrument passes are accurately and unambiguously displayed. Thus, the information provided by the slice is complementary with the information of the projection. The composite 2D image provides information that cannot be presented with only a projection since it misses local information or only a standard plain slice since it misses overall representation.

When the user moves the instrument, the user is always correctly oriented with regard to the patient because of the overall representation and the user can adjust precisely the position of the instrument by using the local information contained in the portion of the slice.

The width of the strip (portion) of the slice can be adjusted interactively. Or it can be adjusted automatically. In a preferred embodiment, the width w of the strip is small when the instrument is far from an expected target position, and larger when the real instrument is approaching the target position.

The same representation can be used when the instrument is inserted through a guide held by a robot, or directly by the robot. The composite 2D image of the invention helps the surgeon to control the overall position of the tool inserted in the robot, for example to check if it is on the desired bone segment, and at the same time to control its precise location.

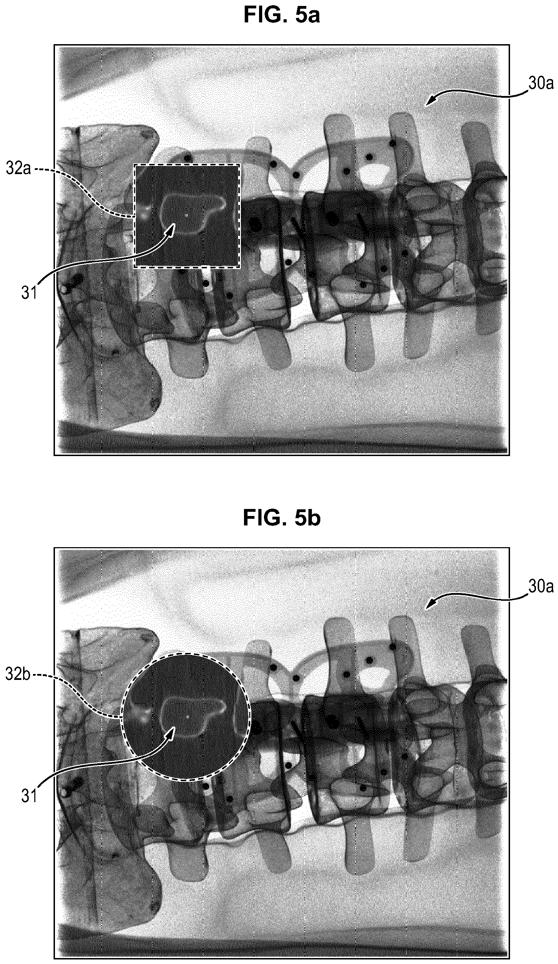

**Figures 5a and 5b** illustrate other examples of composite 2D images that can be obtained. These images 30a, 30b show the axis 31 of the instrument according to the same orientation. In these composite 2D images 30a, 30b, the direction of the projection is merged with the axis of the instrument to provide a plunging perspective of the anatomical region while keeping accurate information of the structures aimed to or traversed by the instrument in a strip. These composite 2D images differ from each other by the shape of the portion of the slice 32a, 32b: a square on figure 5a and a circle on figure 5b.

The method described in the invention can be also used before performing an intervention to facilitate the surgical planning of instruments such as pedicle screws. In conventional planning software, the user navigates the virtual tools in slices determined in several planes. Usually two planes passing through the instrument axis are calculated and displayed. It may happen that the user is lost and does not find the target area because the information is very local. Using the method proposed in the invention, a combined image presenting an overall projection and a small portion of a slice around the tool axis is very helpful to facilitate and speed up the surgical planning.

## Claims

1. Computer implemented method for processing images comprising the following steps:
a) obtaining (100) a 3D image (I-3D) of a region of interest (ROI) including an anatomical region of a patient;
b) obtaining (200) at least one projection (P1) of the region of interest (ROI) of the patient (3), said projection (P1) being a 2D overall image of said region of interest (ROI) and showing information of a first type about said region according to a point of view (Pov);
c) obtaining (300) at least one slice (S) from the 3D image (I-3D), said slice (S) comprising a point of interest (POI) in said region of interest (ROI), said obtained slice containing information about the region of interest (ROI) of a second type around the point of interest (POI);
d) computing (400) a composite 2D image wherein a portion (p) of one of the 2D overall image containing information about the point of interest (POI) is replaced by the projection of a corresponding portion of the obtained slice (S) from the same point of view (Pov) so that information of the second type is accurately arranged relatively to information of the first type present in the 2D overall image.

2. Method according to claim 1, wherein the slice (S) is oriented along a plane orthogonal to a direction of the projection.

3. Method according to claim 1, wherein the slice (S) is parallel to and at a fixed distance from a plane (p11) comprising a display configured to display the composite 2D image, the point of view being included in the plane comprising the display said display being tracked during its movement.

4. Method according to any one of the preceding claims, comprising a step of tracking (800) a medical instrument into the region of interest, said instrument extending along a main axis, the obtained slice being a slice of the region of interest wherein the medical instrument extends.

5. Method according to the preceding claim, wherein the slice (S) passes through the main axis of the medical instrument, wherein the 3D image is associated with a reference frame comprising three cartesian axes X, Y, Z and wherein the slice (S) passes through one of these cartesian axes, such that the slice contains the instrument main axis and has a direction parallel to the chosen cartesian axis.

6. Method according to any one of claims 1 to 3, comprising a step of tracking (800) a medical instrument into the region of interest, said instrument extending along a main axis and wherein the portion (p) of the slice has a width (w) depending on a distance between the instrument main axis and a target point and/or a height defined along the main axis of the instrument.

7. Method according to the preceding claim, wherein the portion (p) of the obtained slice is a strip delimited around the main axis of the instrument.

8. Method according to any one of claims 1 to 3, comprising a step of tracking (800) a medical instrument into the region of interest, said instrument extending along a main axis and wherein the obtaining (300) of the slice (S) comprises the determination of a plane in which the instrument main axis extends.

9. Method according to any one of claims 4 to 8, wherein the point of interest (POI) belongs to the main axis of the medical instrument, the region of interest corresponding to a region in which the medical instrument has to be inserted or has been inserted according to this axis.

10. Method according to any one of claims 4 to 9, wherein the computation (400) of the composite 2D image comprises the projection on said composite 2D image, of either the main axis of the medical instrument the slice being centered around this main axis, or the projection of medical intervention targets.

11. Method according to any one of the preceding claims, comprising a step of selecting (200') a projection among a plurality of projections, each projection being acquired or computed from the 3D image,
- said selection depending on a point of view, said point of view preferably depending on position and/or orientation of a tracked device such as a medical instrument relative to the region of interest or an anatomical feature, or
- said selection depending on the point of interest or on the region of interest.

12. Method according to any one of the preceding claims, wherein the 2D overall image is chosen from the following group: a radiographic image obtained by emission of x-ray to the anatomical region, a Digitally-Reconstructed Radiography, a computed Maximum Intensity Projection, a computed Average Intensity Projection, possibly with enhanced contrast, a computed Minimum Intensity Projection, a computed Locally Minimum, respectively Maximum, Intensity Projection.

13. Method according to any one of the preceding claims, comprising a step of switching between two points of view (Pov), said method implementing the steps b) and d) for taking into account this point of view.

14. Method according to any one of the preceding claims, comprising a step of switching between two slices, said method implementing the steps c) and d) for taking into account the second slice.

15. Method according to any one of claims 13 or 14, wherein the switching is triggered when a relation between a main axis of a tracked device and the direction of the selected projection is below or equal to a defined threshold.

16. Method according to claim 15, wherein the threshold is defined as an angle formed between said main axis of the tracked device and said direction is comprised in a range defined between 35° and 45°.

17. Method according to any one of the preceding claims, comprising a step of displaying the composite image.

18. Medical navigation system comprising a processor configured for implementing the method of any one of the preceding claims.
